**Europäisches Patentamt**

**European Patent Office**

**Office européen des brevets**

(19)

(11) Veröffentlichungsnummer: **0 157 243**
A2

# (12) EUROPÄISCHE PATENTANMELDUNG

(21) Anmeldenummer: **85102870.4**

(22) Anmeldetag: **13.03.85**

(51) Int. Cl.⁴: **G 01 N 21/78**

(30) Priorität: **04.04.84 CH 1698/84**

(43) Veröffentlichungstag der Anmeldung: **09.10.85**
**Patentblatt 85/41**

(84) Benannte Vertragsstaaten: **AT BE CH DE FR GB IT LI NL SE**

(71) Anmelder: **CERBERUS AG, Alte Landstrasse 411, CH-8708 Männedorf (CH)**

(72) Erfinder: **Forster, Martin, Sonnenbergstrasse 16, CH-8645 Jona (CH)**

(74) Vertreter: **Tiemann, Ulrich, Dr.-Ing. et al, c/o Cerberus AG Patentabteilung Alte Landstrasse 411, CH-8708 Männedorf (CH)**

(54) Verfahren und Vorrichtung zum Nachweis von reduzierenden Gasen.

(57) Reduzierende Gase, vorzugsweise Kohlenmonoxid können in einem Gasgemisch, insbesondere Luft, mit außerordentlich hoher Empfindlichkeit und großer Genauigkeit mit einem Gasdetektor (100) nachgewiesen werden, bei welchem die Intensitätsschwächung eines Infrarotstrahls (69) an einer Katalysatorschicht (14) aus einem Metall aus einer der Gruppen I, II, VII und VIII des Periodensystems der chemischen Elemente mit einem Atomgewicht zwischen 100 und 205 ausgenutzt wird. Besonders empfindlich ist der Nachweis von Kohlenmonoxid an einer Katalysatorschicht aus einem Metall, das mit Kohlenmonoxid eine Carbonylverbindung zu bilden vermag. Die Empfindlichkeit und Genauigkeit des Nachweises von Kohlenmonoxid und der anderen reduzierenden Gase kann dadurch beträchtlich erhöht werden, daß das zu untersuchende Gasgemisch in der Meßkammer (7) periodisch ausgetauscht und durch reines Referenzgas aus einer Referenzkammer (5) ersetzt wird; daß während dieser Gasaustauschperiode (27) die Temperatur der Katalysatorschicht (14) periodisch verändert wird und daß das am Ausgang des Gasdetektors (100) erhaltene Wechselsignal zur Bestimmung der Konzentration an reduzierenden Gasen ausgewertet wird.

Verfahren und Vorrichtung zum Nachweis
von reduzierenden Gasen

---

Die Erfindung betrifft ein Verfahren zum Nachweis von reduzierenden Gasen, vorzugsweise von Kohlenmonoxid in einem Gasgemisch, insbesondere in Luft, durch Bestimmung der Intensitätsschwächung eines Infrarotstrahls in einem eine Infrarotstrahlungsquelle, einen Infrarotstrahlungsdetektor und eine
elektrische Auswerteschaltung aufweisenden Gasdetektor und
eine Vorrichtung zur Durchführung dieses Verfahrens.

Vorrichtungen zum Nachweis von Verbindungen, die elektromagnetische Strahlung, insbesondere Infrarotstrahlung, bestimmter Frequenzen bevorzugt absorbieren, sind aus der Spektroskopie allgemein bekannt. Diese Vorrichtungen sind jedoch
ausserordentlich aufwendig und daher in Gaswarnanlagen nicht
zu verwenden. Für Zwecke des Umweltschutzes, Garagenüberwachung, Brandschutz, Explosionsschutz und ähnliche Aufgaben werden daher seit einiger Zeit Gasdetektoren verwendet, wie billige Gassensoren, wie Pellistoren oder Metalloxidhalbleiter,
deren elektrische Leitfähigkeit sich bei der Einwirkung bestimmter Gase verändert, verwendet. Zur Messung der bei der
Einwirkung nachzuweisender Gase auftretenden Leitfähigkeitsänderung müssen die Gassensoren eine bestimmte Mindestgrösse aufweisen und auf eine verhältnismässig hohe Temperatur erhitzt werden. Dadurch bedingt ist die elektrische Leistungsaufnahme dieser Gassensoren relativ hoch. Dies schränkt
ihre Anwendbarkeit in Gaswarnanlagen ein, da die erforderlichen Notstromaggregate entsprechend gross dimensioniert werden müssen.

Es sind daher bereits Versuche unternommen worden, die Veränderung optischer Eigenschaften von Metalloxidhalbleitern bei Einwirkung bestimmter Gase zu deren Nachweis auszunutzen.

Aus der Literatur ist bekannt, dass beispielsweise Rhodium, feinverteilt auf Aluminiumoxid, bei der Einwirkung von gasförmigem Kohlenmonoxid einen sehr hohen Infrarotextinktionskoeffizienten im Bereich der Kohlenmonoxidbande des gasförmigen Kohlenmonoxids aufweist. Nach J. Chem. Phys. $\underline{74}$ (07), 1. April 1981, S.4150 - 4155, ist der Extinktionskoeffizient bei 1950 - 2150 $cm^{-1}$ von $Rh(CO)_x$ etwa 35 mal grösser als von gasförmigem Kohlenmonoxid. Nach J. Chem. Phys. $\underline{74}$ (11), 1. Juni 1981, S. 6487 - 6497, ist $Rh(CO)_x$ auch in Gegenwart von Sauerstoff so stabil, dass es sich bei Zimmertemperatur bilden kann. Zum Nachweis von reduzierenden Gasen, wie von Kohlenmonoxid wurde dieser Effekt in Gaswarnanlagen nicht angewendet.

Die Erfindung hat sich die Aufgabe gestellt, ein einfaches Verfahren zum Nachweis von reduzierenden Gasen, vorzugsweise von Kohlenmonoxid in einem Gasgemisch zu schaffen, das gegenüber den bisherigen Verfahren mittels Infrarotstrahlung erheblich vereinfacht ist und gegenüber den Verfahren mittels Leitfähigkeitsänderung von Metalloxidhalbleitern empfindlicher ist, weniger elektrische Leistung benötigt und keinerlei Nullpunktsdrift aufweist. Ferner soll der Gasdetektor zur Verminderung der Heizleistung kleinere geometrische Abmessungen aufweisen.

Dies wird bei einem Verfahren zum Nachweis von reduzierenden Gasen, vorzugsweise von Kohlenmonoxid, in einem Gasgemisch durch Bestimmung der Intensitätsschwächung eines Infrarotstrahls in einem eine Infrarotstrahlungsquelle, einen Infrarotstrahlungsdetektor und eine elektrische Auswerteschaltung aufweisenden Gasdetektor erfindungsgemäss dadurch erreicht, dass ein Gasdetektor verwendet wird, der eine gegen die Aussenatmosphäre abgeschlossene, einen Luftbewegungsgenerator
aufweisende Referenzkammer und eine mit der Referenzkammer
über mindestens eine Verbindungsöffnung in Verbindung stehende, eine Einlassöffnung zum Einlass des zu untersuchenden
Gasgemischs und einen durch eine Heizung auf eine bestimmte
Temperatur erhitzbaren, mindestens ein Uebergangsmetall aus
einer der Gruppen I, II, VII und VIII des Periodensystems
der chemischen Elemente mit einem Atomgewicht zwischen 100
und 205 enthaltenden Gassensor aufweisende Messkammer aufweist, dass das zu untersuchende Gasgemisch periodisch während einer Einsaugphase durch Volumenvergrösserung der Referenzkammer mittels des Luftbewegungsgenerators durch die
Einlassöffnung in die Messkammer und mindestens teilweise
durch diese hindurch in die Referenzkammer gesaugt wird,
wobei die Temperatur des Gassensors während eines Teils der
Einsaugphase so hoch ist, dass gegebenenfalls vorhandene
reduzierende Gase oxidiert werden und während des Rests der
Einsaugphase so niedrig ist, dass reduzierende Gase am Gassensor adsorbiert werden, so dass ein Referenzgas mit keinem
oder einem geringeren Gehalt an reduzierenden Gasen durch
die Verbindungsöffnung in die Referenzkammer gelangt, dass
das Referenzgas periodisch während einer Ausblasphase durch
Volumenverminderung der Referenzkammer mittels des Luftbewegungsgenerators durch die Messkammer hindurch durch die
Einlassöffnung ausgeblasen wird, wobei die Temperatur des
Gassensors während eines Teils der Ausblasphase annähernd
die gleiche Temperatur aufweist, bei der während der Einsaugphase Oxidation der reduzierenden Gase erfolgte und während
des Rests der Zeit der Ausblasphase annähernd die Temperatur
aufweist, bei der während der Einsaugphase Adsorption von
reduzierenden Gasen erfolgte, dass die Intensität eines von
der Infrarotstrahlungquelle ausgehenden Infrarotstrahls,
der durch den Gassensor hindurchtritt und dann als Messstrahl
auf den Infrarotstrahlungsdetektor fällt, in der elektrischen
Auswerteschaltung ermittelt wird, dass der während der Einsaugphase in, bevorzugt am Ende, der Zeit während der der Gassensor eine Temperatur aufweist, bei der reduzierende Gase adsorbiert werden, gemessene Wert B1 der Intensität des Mess- strahls und der während der Ausblasphase in, bevorzugt am Ende, der Zeit während der der Gassensor eine Temperatur aufweist, bei der reduzierende Gase adsorbiert werden, gemessene Wert B2 der Intensität des Messstrahls zur Bestimmung der Konzentration an reduzierenden Gasen ausgewertet werden.

Die Auswerteschaltung kann so ausgelegt werden, dass bei einer bestimmten Konzentration an reduzierenden Gasen ein Alarm ausgelöst wird, wobei die Auswerteschaltung auch so ausgelegt werden kann, dass bei einer bestimmten geringeren Konzentration an reduzierenden Gasen ein Voralarm (Einschaltung von Absaugvorrichtung, Vorwarnung von Menschen) und bei einer höheren Konzentration an reduzierenden Gasen, z.B. Kohlenmonoxid, ein Hauptalarm (Evakuierung von Menschen, Auslösung von Halonlöschanlagen, Brandalarm) ausgelöst wird. Das erfindungsgemässe Verfahren kann sowohl zur Gasdetektion in Gaswarnanlagen als auch zur Detektion von Verbrennungsprodukten in Brandmeldeanlagen angewendet werden.

Gemäss einer bevorzugten Ausführungsform des erfindungsgemässen Verfahrens wird in dem Gasdetektor ein Gassensor verwendet bei dem mindestens eines der Metalle Rhodium, Rhenium, Iridium, Platin, Palladium, Osmium, Ruthenium, Silber oder Gold auf einem Träger aufgebracht sind, der mindestens eines der Oxide von Magnesium, Calcium, Strontium, Barium, Lathan, Cer, Thorium, Titan, Zirkon, Vanadium, Niob, Tantal, Chrom, Molybdän, Wolfram, Mangan, Eisen, Kobalt, Nickel, Kupfer, Zink, Cadmium, Aluminium, Indium, Silicium, Zinn, Blei, Antimon oder Wismut enthält. Die Metalle liegen bevorzugt in feinst verteilter Form vor, sie können aber auch als dünner Metallfilm ausgebildet sein.

Gemäss einer weiteren bevorzugten Ausführungsform des erfindungsgemässen Verfahrens wird in dem Gasdetektor modulierte Infrarotstrahlung verwendet, wobei eine phasenempfindliche Detektion angewendet wird. Gemäss einer anderen bevorzugten Ausführungsform wird zum Nachweis von Kohlenmonoxid eine Katalysatorschicht aus Rhodium auf Aluminiumoxid verwendet, wobei man in Strahlungsrichtung hinter der Infrarotstrahlungsquelle und/oder vor dem Strahlungsdetektor ein Infrarotfilter mit einem Durchlässigkeitsbereich von 1900 bis 2200 $cm^{-1}$ anbringt. Gemäss weiteren bevorzugten Ausführungsformen werden zum Nachweis von Aethin ein Palladium-auf-Siliziumdioxid-Katalysator und ein Infrarotfilter mit einem Durchlässigkeitsbereich von 2950 bis 3150 $cm^{-1}$ verwendet. Weitere Katalysatoren und Infrarotfilter sind die folgenden: Zum Nachweis von Aethen: Palladium/Siliziumdioxid - Filter 2800 bis 3100 $cm^{-1}$; für Kohlenmonoxid: Rhodium/Aluminiumoxid - Filter 1900 bis 2200 $cm^{-1}$, Palladium/Siliziumdioxid - Filter 1850 bis 2100 $cm^{-1}$ oder Platin/Siliziumdioxid - Filter 1950 bis 2150 $cm^{-1}$; für Aethen: Platin/Siliziumdoixid - Filter 2750 bis 3050 $cm^{-1}$, für Stickstoffoxid (NO) - Rhodium/Aluminiumoxid - Filter 1600 bis 1900 $cm^{-1}$.

Als weitere Trägerstoffe können ausser Aluminium- ($Al_2O_3$) und Siliziumdioxid ($SiO_2$) Zinndioxid ($SnO_2$), Magnesiumoxid (MgO), Zinkoxid (ZnO) und Titandioxid ($TiO_2$) dienen.

Im folgenden werden an Hand der Figuren bevorzugte Ausführungsformen der Erfindung näher erläutert. Es zeigen:

Fig. 1    die schematische Darstellung eines Querschnitts durch einen in dem erfindungsgemässen Verfahren angewendeten Gasdetektor,

Fig. 2    die Intensität des Messstrahls eines Gassensors
          in Abhängigkeit von der Zeit, bzw. den Heizzyklen
          und den Austauschperioden,

Fig. 3    eine spezielle Anordnung eines Gasdetektors für
          den Nachweis eines weiteren Gases neben Kohlenmonoxid,

Fig. 4    eine Gasdetektoranordnung mit einem Hohlspiegel,

Fig. 5    eine Ausgestaltung des erfindungsgemässen Gasdetektors mit beheiztem Hohlspiegel,

Fig. 6    eine Ausgestaltung eines Gasdetektors gemäss Fig.4
          mit verschiedenen IR-Detektoren und mit IR-Filtern
          unterschiedlichen Durchlassbereichs,

Fig. 7    eine Ausgestaltung eines Gasdetektors, bei welcher
          der Gassenosr 11 vom Infrarotstrahl durchdrungen
          wird,

Fig. 8    eine Weiterbildung eines GAsdetektors gemäss Fig.7,
          bei welcher eine Kollimatorlinse den Infrarotstrahl
          bündelt,

Fig. 9    eine weitere Ausgestaltung eines Gasdetektors gemäss

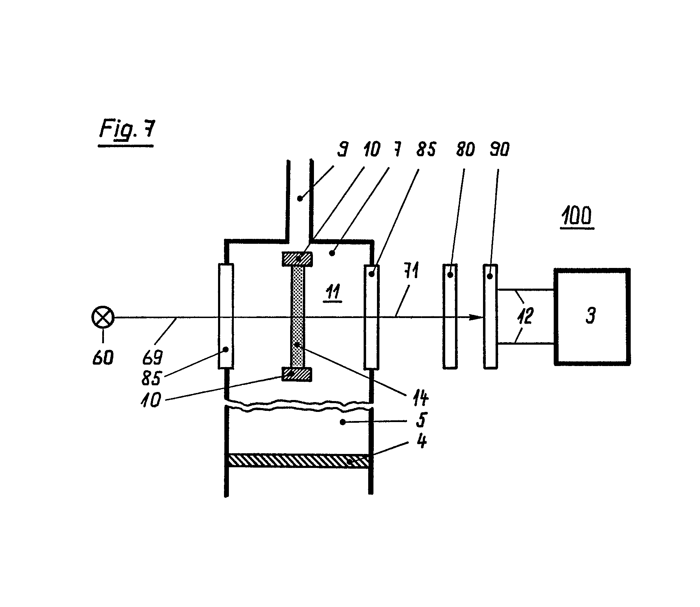

          Fig.7, bei welcher der Infrarotstrahl mit einem
          Hohlspiegel gebündelt wird,

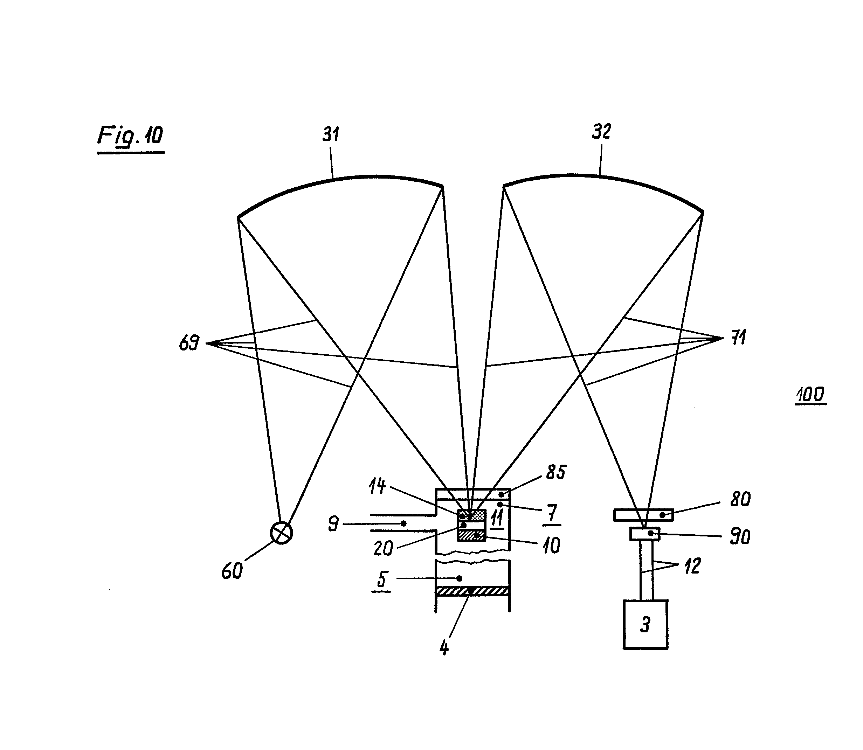

Fig. 10   eine weitere Ausgestaltung eines Gasdetektors mit
          zwei Hohlspiegeln und

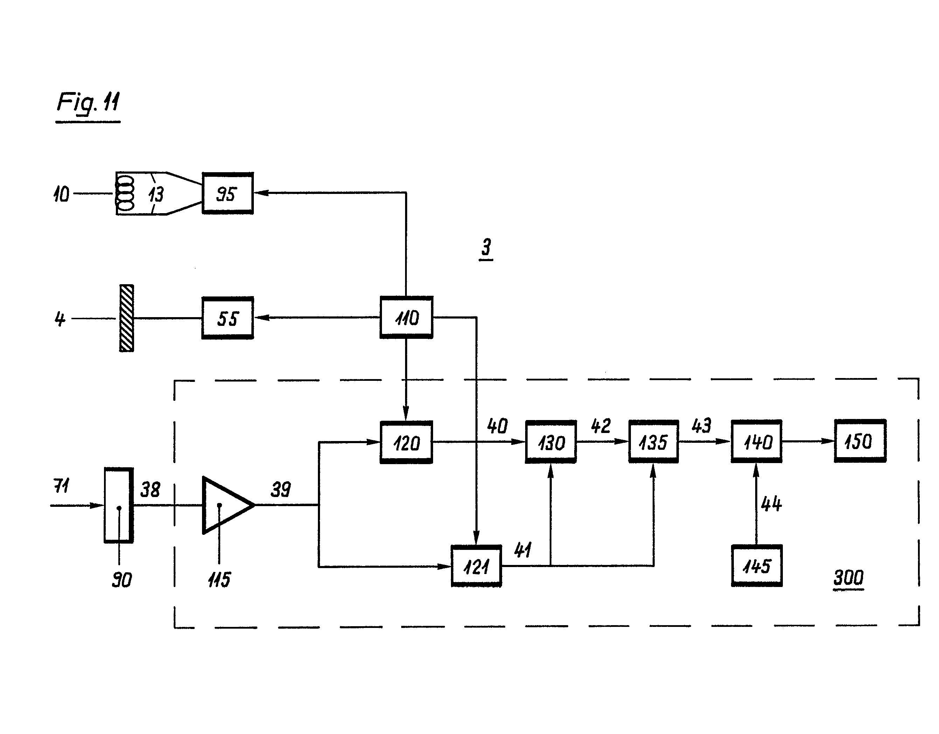

Fig. 11   eine mögliche Auswerteschaltung.


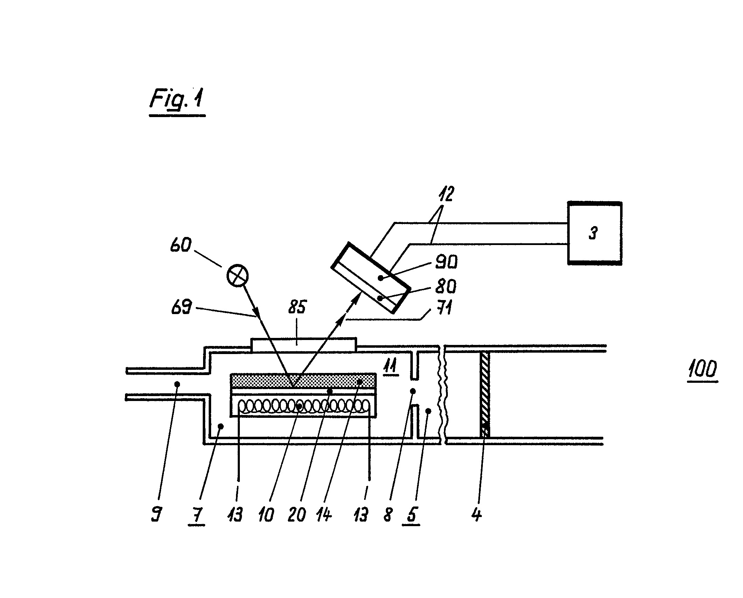

Das in Fig. 1 dargestellte Schema eines Gasdetektors 100
besteht im wesentlichen aus einer Messkammer 7 und einer
gegenüber der Aussenatmosphäre abgeschlossenen Referenzkammer 5. Die Messkammer 7 ist dabei zur besseren Erkennbarkeit der Details in einem grösseren Massstab dargestellt. In der Messkammer 7 befindet sich der Gassensor 11 mit einer Katalysatorschicht 14, der von der Heizung 10 auf eine bestimmte Temperatur aufgeheizt werden kann. Der Heizung 10 wird durch die elektrische Leitung 13 von einer nicht dargestellten Energiequelle elektrische Energie zugeführt.

Durch die Einlassöffnung 9 wird der Messkammer 7 das zu untersuchende Gasgemisch zugeführt. Ueber die Verbindungsöffnung 8 ist die Messkammer 7 mit der Referenzkammer 5, in der ein Luftbewegungsgenerator 4 angeordnet ist, verbunden. In eine Seitenwand der Messkammer 7, die ausser der Einlassöffnung 9 gegen die Aussenatmosphäre hermetisch abgeschlossen ist, ist ein Infrarotfenster 85, d.h. ein für Infrarotstrahlung bestimmter Frequenz durchlässiges Fenster, eingelassen, durch welches der Infrarotstrahl 69 einer Infrarotstrahlungsquelle 60 in die Messkammer 7 eindringen und auf den Gassensor 11 fallen kann. Der Infrarotstrahl 69 durchdringt die Katalysatorschicht 14 des Gassensors 11, wird von der unterhalb des Gassensors 11 angebrachten reflektierenden Schicht 20 reflektiert, durchdringt ein zweites Mal die Katalysatorschicht 14 des Gassensors 11 und verlässt als Messtrahl 71 durch das Infrarotfenster 85 die Messkammer 7. Der Messstrahl 71 fällt durch das Infrarotfenster 80 auf den Infrarotstrahlungsdetektor 90.

Ueber die Leitungen 12 ist der Infrarotstrahlungsdetektor 90 mit einer Auswerteschaltung 3 verbunden, welche die mittels des Infrarotstrahlungsdetektors 90 gemessene Intensität I des Messstrahls 71 zur Bestimmung der Konzentration an Kohlenmonoxid auswertet und gegebenenfalls ein Alarmsignal abgibt. Die Auswerteschaltung 3 enthält ausserdem Schaltungselemente, durch welche die Bewegung des Luftbewegungsgenerators 4 und die Temperatur des Gassensors 11 mittels
der Heizung 10 gesteuert werden. Diese Schaltungselemente
und die erforderlichen Verbindungsleitungen sind nicht dargestellt.

Als Infrarotstrahlungsquelle 60 dient ein Glühlämpchen oder
ein handelsüblicher Nernst-Stift. Das Infrarotfenster 85
besteht aus einem für Infrarotstrahlen durchlässigen Material. Dem Infrarotstrahlungsdetektor 90 ist ein Infrarotfenster 80 vorgeschaltet, das Infrarotstrahlung im Gebiet von
1900 bis 2200 $cm^{-1}$ durchlässt. Als Luftbewegungsgenerator
4 dient ein handelsüblicher miniaturisierter Lautsprecher.

Der Gassensor 11 enthält eine Katalysatorschicht 14, die
aus auf Aluminiumoxid niedergeschlagenem feinstverteilten
Rhodium besteht und für Infrarotstrahlung im Bereich 1900
bis 2200 $cm^{-1}$ durchlässig ist. Unterhalb dieser Katalysatorschicht 14 befindet sich eine reflektierende Schicht 20 aus
Gold oder einem anderen Edelmetall, an welcher der Infrarotstrahl 69 reflektiert wird.

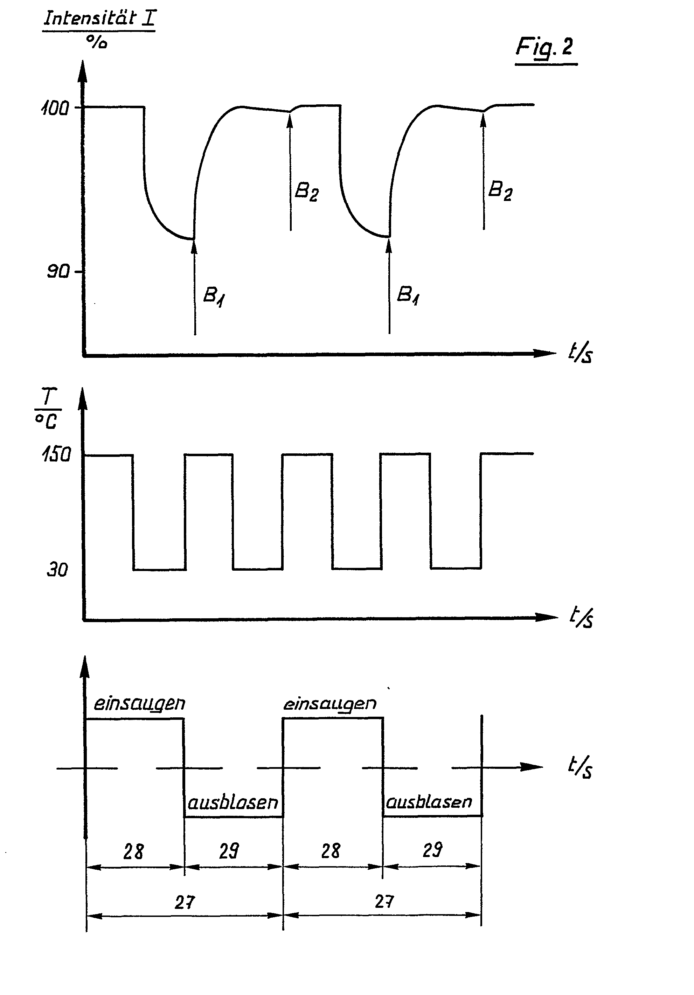

Im folgenden wird die Arbeitsweise eines in Fig. 1 schematisch dargestellten Gasdetektors 100 anhand der Fig. 2
beschrieben. Der Gassensor 11 wird mittels Heizung 10
auf eine Temperatur erhitzt, bei welcher die Katalysatorschicht 14 des Gassensors 11 in der Lage ist, die Oxidation von  Kohlenmonoxid zu Kohlendioxid zu katalysieren beispielsweise auf eine Temperatur von ca. 150°C (Oxidationstemperatur). Die Entfernung des Kohlenmonoxids muss nicht unbedingt durch Oxidation erfolgen, sie kann auch durch andere chemische Umsetzungen oder durch Herauspumpen mittels Ionenpumpe erfolgen. Entfernung durch reine Adsorption genügt aber jedenfalls in dieser Phase des Verfahrens nicht. Der Einfachheit halber wird aber von "Oxidations"-Temperatur gesprochen.

Gleichzeitig wird durch eine Volumenvergrösserung der Referenzkammer 5 mittels des Luftbewegungsgenerators 4 das zu untersuchende Gasgemisch durch die Einlassöffnung 9 in die Messkammer 7 und teilweise durch diese hindurch durch die Verbindungsöffnung 8 in die Referenzkammer 5 gesaugt. Während die Periode des Einsaugens 28 fortgesetzt wird, wird die Temperatur der Katalysatorschicht 14 des Gassensors 11 auf eine Temperatur abgesenkt, die so niedrig ist, dass gegebenenfalls in der zu untersuchenden Gasmischung vorhandenes Kohlenmonoxid vom Gassensor 11 nicht oxidiert, sondern adsorbiert wird (Adsorptionstemperatur). Eine solche Temperatur beträgt beispielsweise 30°C. Nach Beendigung der Periode des Einsaugens 28 wird durch Volumenverminderung der Referenzkammer 5 mittels des Luftbewegungsgenerators 4 das Kohlenmonoxid in geringerer Konzentration enthaltende, bzw. von Kohlenmonoxid befreite Referenzgas durch die Verbindungsöffnung 8 in die Messkammer 7 und durch diese hindurch durch die Einlassöffnung 9 aus dem Gasdetektor 100 ausgeblasen. Während des Anfangs dieser Periode des Ausblasens 29 ist die Temperatur des Gassensors 11 annähernd gleich derjenigen, bei der während des Einsaugens 28 Oxidation von gegebenenfalls vorhandenem Kohlenmonoxid erfolgte, d.h. etwa 150°C. Während des Restes der Zeit der Periode des Ausblasens 29 wird die Temperatur des Gassensors 11 annähernd auf die Temperatur abgesenkt, bei der während der Periode des Einsaugens 28 Adsoption von gegebenenfalls vorhandenem Kohlenmonoxid erfolgte, d.h. ca. 30°C. Diese aus Einsaugen 28 und Ausblasen 29 bestehende Austauschperiode 27 wird nun ständig wiederholt, wobei die Temperatur des Gassensors 11 ebenfalls periodisch zwischen der Oxidationstemperatur, z.B. 150°C, und der Adsorptionstemperatur, z.B. 30°C, variiert.

Entsprechend der variierenden Transmission der Katalysatorschicht 14 des Gassensors 11 im Bereich von 1900 - 2200 cm$^{1}$ während dieser Austauschperioden 27 verändert sich auch die Intensität I des Messstrahles 71, die vom Infrarotstrahlungsdetektor 90 gemessen wird. In Fig. 2 ist die Intensität I des Messstrahles 71 in Prozenten als Funktion der Zeit t aufgetragen.

Zu Beginn der Periode des Einsaugens 28, während der der Gassensor 11 auf der Oxidationstemperatur gehalten wird, beträgt die Intensität I des Messstrahls 100%. Beim Absenken der Temperatur auf die Adsorptionstemperatur von ca. 30°C sinkt die Transmission der Katalysatorschicht 14 durch Adsoption von Kohlenmonoxid, und die Intensität I des Messstrahles 71 erreicht Punkt $B_1$. Sobald die Temperatur nach Beginn des Ausblasens 29 wieder auf die Oxidationstemperatur von 150°C erhöht wird, steigt auch die Transmission der Katalysatorschicht 14 und damit die Intensität I des Messstrahles 71 wieder auf den alten Wert von 100%, da das auf der Katalysatorschicht 14 adsorbierte Kohlenmonoxid oxidiert wird.

Beim weitern Ausblasen und Absenken der Temperatur des Gassensors 11 auf die Adsorptionstemperatur von ca. 30°C sinkt die Transmission der Katalysatorschicht 14 und damit die Intensität I des Messstrahls 71 nur noch wenig auf den Wert $B_2$, da nun aus der Reservekammer 5 von Kohlenmonoxid befreites oder nur noch Spuren von Kohlenmonoxid enthaltendes Gasgemisch über den Gassensor 11 geleitet wird.

0157243

Die vom Infrarotstrahlungsdetektor 90 erzeugten, der Intensität des Messstrahls 71 entsprechenden elektrischen Signale werden an die Auswerteschaltung 3 gegeben. In der Zeit des Einsaugens 28, während der der Gassensor 11 eine Temperatur aufweist, bei der die Katalysatorschicht 14 Kohlenmonoxid adsorbiert, wird ein Wert $B_1$ gemessen - bevorzugt am Ende der Zeit der Adsorptionstemperatur - und in der Zeit des Ausblasens 29, während der der Gassensor 11 ebenfalls eine Temperatur aufweist, bei der die Katalysatorschicht 14 Kohlenmonoxid adsorbiert, wird ein Wert $B_2$ gemessen - ebenfalls bevorzugt am Ende der Zeit der Adsorptionstemperatur.

Aus diesen beiden Werten $B_1$, $B_2$, die den Intensitäten $B_1$, $B_2$ in Fig. 2 entsprechen, berechnet nun die Auswerteschaltung 3 die Kohlenmonoxidkonzentration [CO], die durch Gleichung (1) gegeben ist

$$[CO] \sim \alpha \ (B_2 - B_1) / B_2 \qquad (1)$$

worin $\alpha$ eine Proportionalitätskonstante ist. Gleichung (1) gibt nur für $(B_2 - B_1) / B_2 \leqslant 0,1$ exakte Resultate, für grössere Werte von $(B_2 - B_1) / B_2$ muss die Berechnung der Kohlenmonoxidkonzentration [CO] nach Gleichung (2) erfolgen, worin $\beta$ eine weitere Proportionalitätskonstante ist

$$[CO] = -\beta \log \frac{B_1}{B_2} = -\beta \ (\log B_1 - \log B_2) \qquad (2).$$

Da der Gasdetektor 100 hauptsächlich zum Nachweis von kleinen CO-Konzentrationen gedacht ist, erfolgt die Bestimmung der CO-Konzentration durch die Auswerteschaltung 3 bevorzugt nach Gleichung (1).

Da die Katalysatorschicht 14, wahrscheinlich durch die Bildung von Rhodiumcarbonylverbindungen, spezifisch auf Kohlenmonoxid reagiert, ist mittels des erfindungsgemässen Verfahrens ein ausserordentlich spezifischer Nachweis von Kohlenmonoxid mit ausserordentlicher Empfindlichkeit geschaffen.

Zur Vermeidung von Störungen kann die Infrarotstrahlungsquelle 60 mit einer Frequenz gepulst werden, die wesentlich grösser ist als die Frequenz der Gasaustauschperiode 27. Alle Signale des Infrarotstrahlungsdetektors 90 werden durch phasenempfindliche Gleichrichtung erfasst, in DC-Signale umgewandelt und durch ein "sample and hold device" an den Punkten $B_1$ und $B_2$ gemessen; die Messwerte werden dann durch einen Analog/Digital-Wandler in Digitalsignale umgewandelt und diese nach Gleichung (1) zur Bestimmung der CO-Konzentration benutzt.

Die Auswerteschaltung 3 ist so ausgelegt, dass sie bei Ueberschreiten bestimmter Konzentrationswerte, die sich aus der durch $B_2$ dividierten Differenz von $B_1$ und $B_2$ oder angenähert auch nur aus $B_2-B_1$ ergeben, einen Alarm auslöst.

Anstelle des modulierten Betriebs der Infrarotstrahlungsquelle 60 kann auch der Infrarotstrahl 69 und/oder der Messstrahl 71 durch einen mechanischen Chopper moduliert werden.

Der Luftbewegungsgenerator 4 kann z.B. als elektromagnetisch, elektrostatisch, piezoelektrisch oder thermomechanisch erregbare Membran in der Art eines Lautsprechers ausgeführt sein oder eine Piezofolie vom Typ PFDF (Polyvinylidendifluorid) aufweisen oder ein bimorphes, piezoelektrisches oder bimetallisches Element enthalten. In miniaturisierten Gasdetektoren kann der Luftbewegungsgenerator 4 auch eine durch Mikrolithographie erzeugte Siliziummembran sein. In einem praktisch ausgeführten Beispiel erwies sich als Luftbewegungsgenerator ein kommerzieller Minilautsprecher als geeignet. Als geeignete Schwingungsfrequenz des Luftbewegungsgenerators erwies sich eine Frequenz zwischen 0,01 und 10 Hz, vorzugsweise etwa 0,1 Hz, als geeignet.

Die Vorteile des erfindungsgemässen Verfahrens gegenüber den bisher bekannten spektroskopischen Verfahren sind unter anderem die folgenden:

1. Dank der z.B. alle 30 Sekunden erfolgenden Vergleichsmessungen in Punkt $B_2$ Ausrechnen der Konzentrationen aus der Differenz von $B_1$ und $B_2$ und Normieren durch die Bildung des Quotienten $(B_2 - B_1)/B_2$ wird jegliche Nullpunktdrift durch Verstauben, Verschmutzen u.ä. aller optischen Komponenten vollständig kompensiert.

0157243

2. Der Gasdetektor 100, insbesondere der Gassensor 11, kann ausserordentlich klein gehalten werden; der Gassensor 11 braucht z.B. die Masse von 2 x 2 x 2mm$^3$ nicht zu übersteigen.

3. Die Messungen sind ausserordentlich empfindlich, da sie ein Prinzip anwenden bei dem am Gassensor 11 perodisch zu untersuchendes Gasgemisch gegen Refernzgas ausgetauscht wird und die Differenz der erhaltenen Signale ausgewertet wird.

4. Da die Katalysatorschicht des Gassensors nur einige mm$^2$ gross sein muss und die Heiztemperatur nicht höher als 150°C betragen muss, ist die Leistungsaufnahme des Gasdetektors 100 ausserordentlich gering.

5. Dank der Beschränkung auf das Spektralgebiet von 1900 - 2200 cm$^{-1}$ ist der Gassensor ausserordentlich selektiv für Kohlenmonoxid.

6. Der Gasdetektor 100 kann auch zur Bestimmung anderer Gase verwendet werden, wenn mindestens ein weiterer Gassensor, der für andere Gase selektiv ist, vorgesehen wird. Diese weiteren Gassensoren können mit der gleichen Infrarotstrahlungsquelle 60 und der gleichen Heizung 10 betrieben werden, wenn mehrere Hohlspiegel und Infrarotstrahlungsdetektoren verwendet werden (kleines Spektroskop für den Brandschutz, Gasschutz, Umweltschutz).

7. Durch Adsorption von Gas an der Katalysatorschicht wird lokal eine sehr hohe Kohlenmonoxidkonzentration im Katalysator erreicht, so dass eine dünne Katalysatorschicht vollständig ausreicht. Durch das Ansaugen wird ständig frisches zu untersuchendes Gasgemisch mit gegebenenfalls vorhandenem Kohlenmonoxid zugeführt.

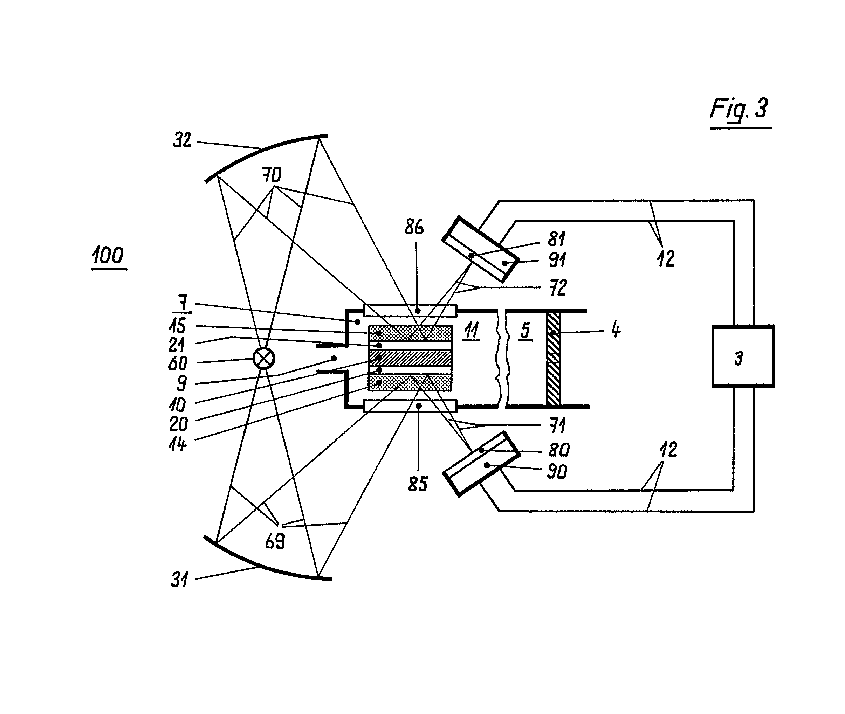

In Fig. 3 ist ein Gasdetektor 100 dargestellt, mit dem neben Kohlenmonoxid ein weiteres Gas nachgewiesen werden kann. Bei diesem Gasdetektor 100 weisen Messkammer 7 und Referenzkammer 5 den gleichen Querschnitt auf, eine Verbindungsöffnung entfällt in diesem Fall. In diesem und gegebenefalls in den folgenden Beispielen kann die Messkammer 7 zur besseren Erkennbarkeit der Details in einem erheblich grösseren Massstab dargestellt sein. Ueber die Einlassöffnung 9 wird dem Gasdektor 100 das zu untersuchende Gasgemisch zugeführt; in der Referenzkammer 5 befindet sich der Luftbewegungsgenerator 4.

Von der Infrarotstrahlungsquelle 60 gehen zwei Infrarotstrahlen aus. Der eine Infrarotstrahl 69 fällt durch ein erstes Infrarotfenster 85 auf die erste Katalysatorschicht 14, wird von der darunter angeordneten ersten reflektierenden Schicht 20 ein zweites Mal durch die erste Katalysatorschicht 14 reflektiert und fällt nach erneutem Durchgang durch das erste Infrarotfenster 85 auf den mit einem ersten Infrarotfilter 80 versehenen ersten Infrarotstrahlungsdetektor 90. Der zweite Infrarotstrahl 70 fällt auf einen zweiten Hohlspiegel 32 durch ein zweites Infrarotfenster 86 auf eine zweite Katalysatorschicht 15; nach Reflektion an einer zweiten reflektierenden Schicht 21 dringt der Infrarotstrahl erneut durch die zweite Katalysatorschicht 15, tritt durch ein zweites Infrarotfenster 86 aus der Messkammer 7 aus und fällt als zweiter Messstrahl 71 auf den mit einem zweiten Infrarotfilter 81 versehenen Infrarotstrahlungsdetektor 91. Wie bei dem in Fig. 1 dargestellten Gasdetektor 100 sind die Infrarotdetektoren 90 und 91 mit einer Auswerteschaltung 3 über elektrische Leitungen 12 verbunden, welche die mittels der Infrarotstrahlungsdetektoren 90 und 91 gemessenen Transmissionswerte der Katalysatorschichten 14 und 15 zur Bestimmung der Konzentration an Kohlenmonoxid und einer weiteren Gaskomponente auswertet und gegebenenfalls ein Alarmsignal abgibt.

Auch hier enthält die Auswerteschaltung 3 Schaltungselemente, durch welche die Bewegung des Luftbewegungsgenerators 4 und die Temperatur des Gassensors 11 mittels der Heizung 10 gesteuert werden. Diese Schaltungselemente und die erforderlichen Verbindungsleitungen sind nicht dargestellt.

Die erste Katalysatorschicht 14, das erste Infrarotfenster 85 und das erste Infrarotfilter 80, das sich vor dem ersten Infrarotstrahlungsdetektor 90 befindet, entsprechen dabei den entsprechenden Teilen des in Fig. 1 dargestellten Gasdetektors 100, d.h. dieser Teil des Gasdetektors 100 ist für den Nachweis von Kohlenmonoxid ausgelegt.

Die zweite Katalysatorschicht 15, das zweite Infrarotfenster 86 und das zweite Infrarotfilter 81, das sich vor dem zweiten Infrarotstrahlungsdetektor 91 befindet, sind dabei für den Nachweis eines weiteren Bestandteils des zu untersuchenden Gasgemischs ausgelegt. Durch bestimmte Wahl des Materials der zweiten Katalysatorschicht 15 und des Spektralbereichs des zweiten Infrarotstrahls 70 - durch Wahl des Materials des zweiten Infrarotfensters 86 und des vor dem zweiten Infrarotstrahlungsdetektor 91 befindlichen zweiten Infrarotfilters 81 - kann beispielsweise Schwefeldioxid nachgewiesen werden.

Durch Wahl einer dritten Katalysatorschicht mit entsprechender Ausgestaltung des Gasdetektors 100 können auch noch weitere gasförmige Bestandteile in dem zu untersuchenden Gasgemisch nachgewiesen werden.

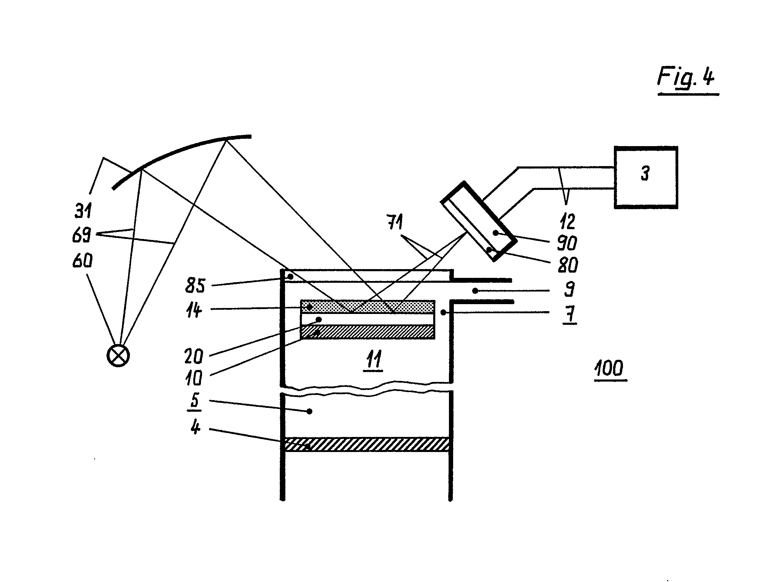

In Fig. 4 ist ein Gasdetektor 100 mit einem Hohlspiegel 31 dargestellt. Von einer Infrarotstrahlungsquelle 60 fällt ein Infrarotstrahl 69 auf einen Hohlspiegel 31, von dem er durch das Infrarotfenster 85 in die Messkammer 7 reflektiert wird. Messkammer 7, Verbindungsöffnung 8 und Referenzkammer 5 haben den gleichen Querschnitt. In der Referenzkammer 5 befindet sich der Luftbewegungsgenerator 4, und die Messkammer 7 ist durch die Einlassöffnung 9 mit der Aussenatmosphäre verbunden. Der Infrarotstrahl 69 wird durch eine unter der Katalysatorschicht 14 befindliche reflektierende Schicht 20 des mit der Heizung 10 versehenen Gassensors 11 nach erneutem Durchtritt durch die Katalysatorschicht 14 und das Infrarotfenster 85 als Messstrahl 71 auf den mit einem Infrarotfilter 80 versehenen Infrarotstrahlungsdetektor 90 geworfen. Die Arbeitsweise dieser Gasdetektoranordnung entspricht der Arbeitsweise des in Fig. 1 dargestellten Gasdetektors 100.

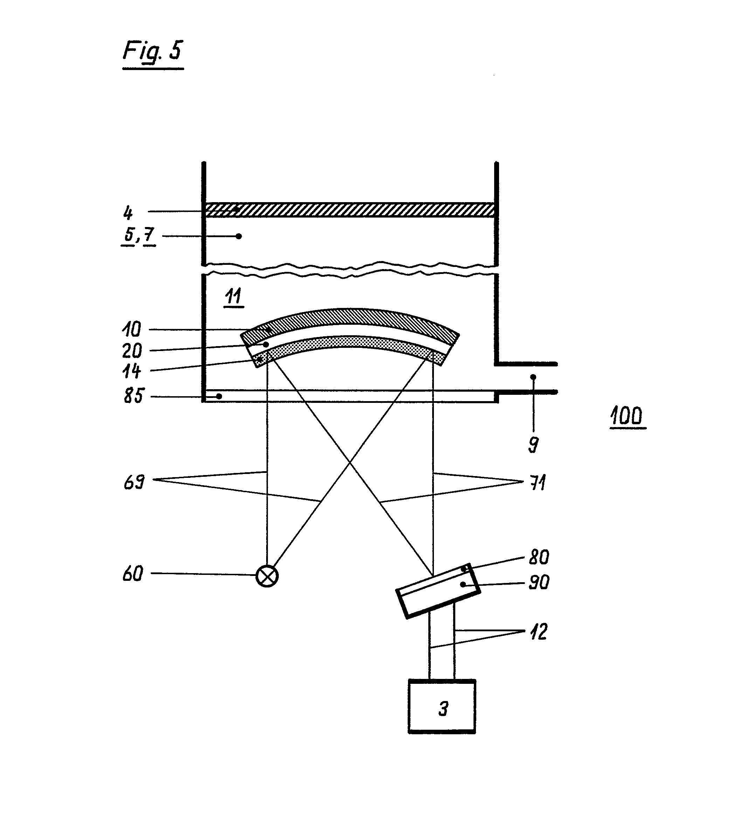

In Fig. 5 ist eine weitere Ausgestaltung des erfindungsgemässen Gasdetektors 100 gezeigt, bei der nicht ein separater Hohlspiegel verwendet wird, sondern bei der die reflektierende Schicht 20 als Hohlspiegel ausgebildet ist. Durch diese Anordnung wird der von der Infrarotstrahlungsquelle 60 ausgehende Infrarotstrahl 69 durch das Infrarotfenster 85 auf die Katalysatorschicht 14 geworfen. Unter der Katalysatorschicht 14 befindet sich die reflektierende Schicht 20, die als Teil einer Kugeloberfläche, eines Ellipsoids oder eines Paraboloids so ausgebildet ist, dass nach Reflektion der Messstrahl 71 durch das Infrarotfenster 85 und des Infrarotfilter 80 auf den Infrarotstrahlungsdetektor 90 fokussiert wird. Die reflektierende Schicht 20 und die Katalysatorschicht 14 werden durch die Heizung 10 auf die erforderlichen Temperaturen gebracht. Die Einlassöffnung 9 ist in diesem Beispiel als eine 1 cm lange Kapillare von 0,5 mm Innendurchmesser ausgebildet. Dadurch wird dieser Gasdetektor 100 völlig unabhängig von der Strömungsgeschwindigkeit des zu untersuchenden Gasgemisches. Die übrigen Teile des Gasdetektors 100 entsprechen den in den vorstehenden Figuren dargestellten Gasdetektoren.

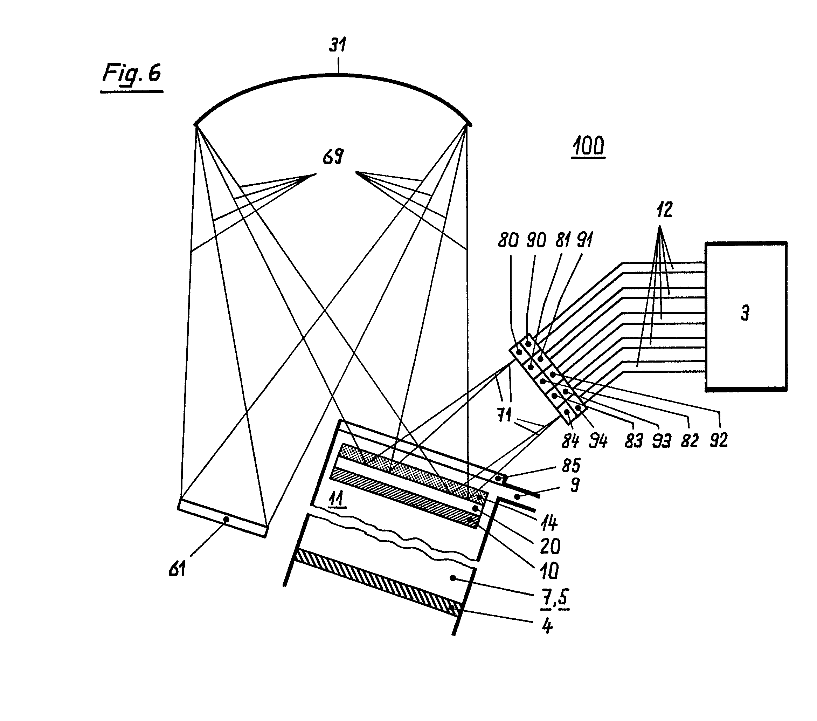

In Fig. 6 ist eine weitere Ausgestaltung eines Gasdetektors gemäss Fig. 4 dargestellt, bei der der Infrarotstrahl 69 durch einen Hohlspiegel 31 nach Reflektion an einer unterhalb der Katalysatorschicht befindlichen, reflektierenden Schicht 20 auf eine Reihe breitbandiger Infrarotstrahlungsdetektoren 90 bis 94 fällt. Als Infrarotstrahlungsquelle dient eine langgestreckte Infrarotstrahlungsquelle 61, die in Verbindung mit dem Hohlspiegel 31 und der langgestreckten Katalysatorschicht 14 so ausgelegt ist, dass der Messstrahl 71 auf die langgestreckt nebeneinander angeordneten, breitbandigen Infrarotstrahlungsdetektoren 90 bis 94 fällt. Vor den breitbandigen Infrarotstrahlungsdetektoren 90 bis 94 sind unterschiedliche, schmalbandige Infrarotfilter 80 bis 84 angeordnet. Die langgestreckte Katalysatorschicht 14 ist aus einheitlichem Material ausgebildet, das sich zur Adsorption verschiedener Gase eignet. Durch die Adsorption unterschiedlicher Gase wird eine unterschiedliche Transmission für Infrarotstrahlung bewirkt. Dadurch dass vor den breitbandigen Infrarotstrahlungsdetektoren 90 bis 94 unterschiedliche schmalbandige Infrarotfilter 80 bis 84 angeordnet sind, die Infrarotstrahlung in unterschiedlichen Wellenlängenbereichen hindurchlassen, ist der Nachweis verschiedener nachzuweisender Gase in dem zu untersuchenden Gasgemisch möglich.

In Fig. 7 ist eine Ausgestaltung eines Gasdetektors 100 dargestellt, bei welcher der Gassensor 11 vom Infrarotstrahl 69 durchdrungen wird. Messkammer 7, Verbindungsöffnung 8 und Referenzkammer 5 haben wiederum den gleichen Querschnitt und die Messkammer 7 steht über die Einlassöffnung 9 mit der Aussenatmosphäre in Verbindung. Zwei gleiche Infrarotfenster 85 befinden sich auf entgegengesetzten Seiten der Messkammer 7, zwischen beiden befindet sich die Katalysatorschicht 11, die aus auf Aluminiumoxid niedergeschlagenem, feinverteiltem Rhenium besteht. Um die Katalysatorschicht 11 herum befindet sich ausserhalb des Strahlenganges des Infrarotstrahls 69 die Heizung 10. Auf der einen Seite der Messkammer 7 befindet sich die Infrarotstrahlungsquelle 60, von welcher der
Infrarotstrahl 69 durch die Katalysatorschicht 11 hindurch
(und durch die beiden Infrarotfenster 85 hindurch) als Messstrahl 71 auf den hinter dem Infrarotfilter 80 befindlichen
Strahlungsdetektor 90 fällt.

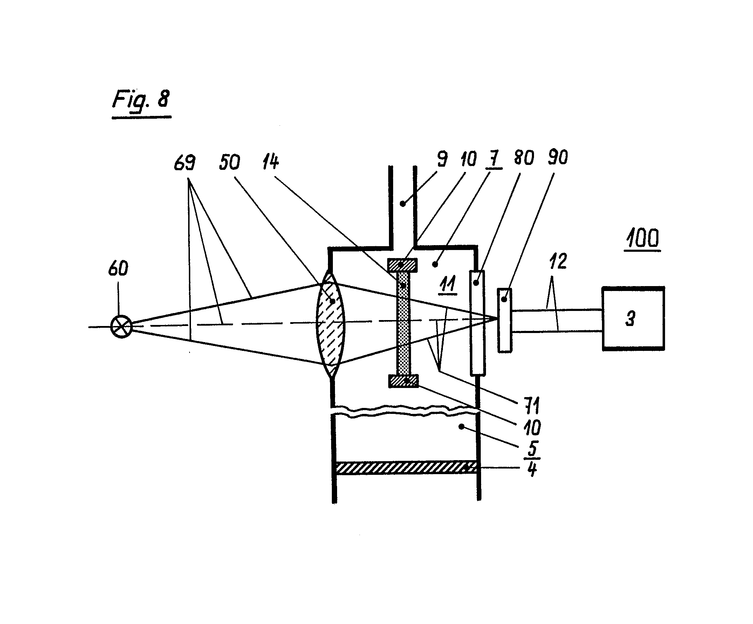

Zur Verbesserung der Strahlungsausbeute - und damit zur Verminderung des Stromverbrauchs - kann zwischen der Infrarotstrahlungsquelle 60 der in der Messkammer 7 befindlichen Katalysatorschicht 14 eine Infrarotlinse 50 angeordnet sein.
Um Reflexionsverluste möglichst klein zu halten, ist die
Linse 50 direkt in der einen Wand und der Infrarotfilter 80
in der andern Wand der Messkammer 7 eingelassen. Dadurch können die Infrarotfenster 85 der vorstehend beschriebenen Ausführungsformen eingespart werden. Der Strahlengang ist in
Fig. 8 dargestellt. Die übrigen Teile des Gasdetektors 100
entsprechen den vorstehend beschriebenen Gasdetektoren 100.

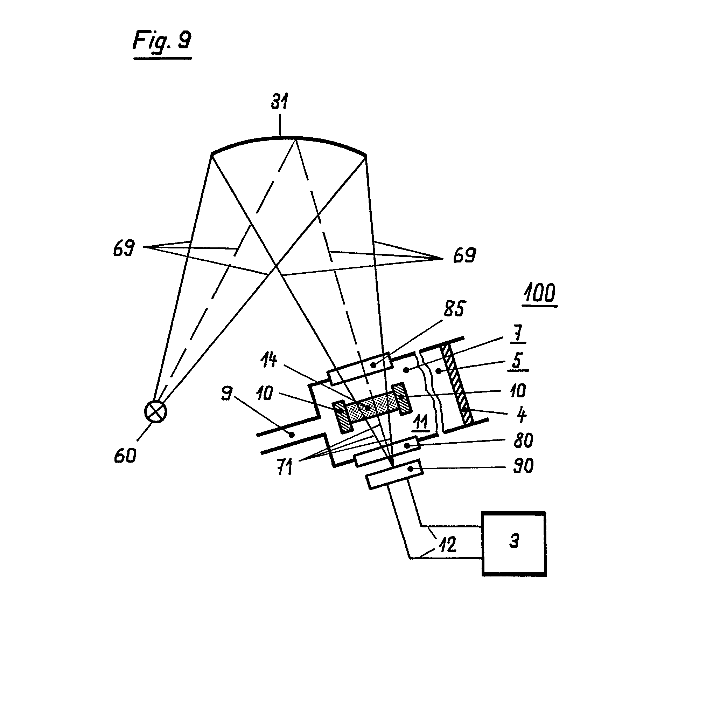

In Fig. 9 ist eine weitere Ausgestaltung eines Gasdetektors
100 gemäss Fig. 7 dargestellt, bei welcher der Infrarotstrahl
69 mit einem Hohlspiegel 31 so gebündelt wird, dass der Messstrahl 71 nach Durchtritt durch die Katalysatorschicht 14
des Gassensors 11 und durch den in der Wand der Messkammer 7
eingelassenen Infrarotfilter 80 auf den Infrarotstrahlungsdetektor 90 fokussiert wird.

In Fig. 10 ist eine weitere Ausgestaltung eines Gasdetektors
100 dargestellt, bei welcher einerseits der Infrarotstrahl
69 auf die Katalysatorschicht 14 das Gassensors 11 fokussiert
wird und andererseits der von der Katalysatorschicht 14 ausgehende Messstrahl 71 auf den Infrarotstrahlungsdetektor 90
fokussiert wird. Der von der Infrarotstrahlungsquelle 60 ausgehende Infrarotstrahl 69 fällt auf einen ersten Hohlspiegel
31, der diesen so fokussiert, dass er durch das Infrarotfenster 85 in den in der Messkammer 7 befindlichen Gassensor 11 fällt. Messkammer 7, Verbindungsöffnung 8 und Referenzkammer 5 haben wie in den vorangehenden Beispielen den gleichen Querschnitt, d.h. der Gasdetektor 100 weist eine gemeinsame Mess- und Referenzkammer auf. Diese steht über die Einlassöffnung 9 mit der Aussenatmosphäre in Verbindung. Der von der Katalysatorschicht 14 des Gassensors 11 ausgehende divergierende Messstrahl 71 fällt auf einen zweiten Hohlspiegel 32, der so ausgebildet ist, dass der Messstrahl 71, der durch das Infrarotfilter 80 hindurchgeht, direkt auf den Infrarotstrahlungsdetektor 90 fokussiert wird. Die Intensität dieses Messstrahls 71 wird in Form eines elektrischen Signals über die elektrischen Leitungen 12 an die Auswerteschaltung 3 gegeben und in üblicher Weise ausgewertet.

Diese Art der Anordnung bringt eine Energieeinsparung mit sich, da die Katalysatorschicht 14 auf dem Gassensor 11 ausserordentlich klein gehalten werden kann.

Auch bei den Ausgestaltungen des Gasdetektors 100 gemäss Figuren 7 bis 10 können durch Anordnung mehrerer Infrarotstrahlungsdetektoren mit unterschiedlichen Infrarotfiltern verschiedene Gasbestandteile in dem zu untersuchenden Gasgemisch nachgewiesen werden.

Fig. 11 zeigt eine mögliche Ausführung der Auswerteschaltung 3, die auf alle in Fig. 1 bis 10 dargestellten Gasdetektoren 100 angewendet werden kann.

Die Synchronisiereinheit 110 steuert über die Heizspannungsquelle 95 den zeitlichen Ablauf des Temperaturzyklus und über den Treiber 55 die Bewegungen des Luftbewegungsgenerators 4. Das vom Infrarotstrahlungsdetektor 90 erzeugte, von der Intensität I des Messstrahls 71 abhängige Signal 38 wird durch den Verstärker 115 - mit einer entsprechenden Verstärkungscharakteristik - in eine der Intensität I des Messstrahles 71 proportionale Spannung 39 umgesetzt.
Die "Sample-and-Hold"-Einheiten 120 und 121, die den Befehl zum Messen von der Synchronisiereinheit 110 erhalten, erzeugen nun Spannungen 40 und 41, die den Werten $B_1$, bzw. $B_2$ in Fig. 2 entsprechen. Im Subtraktionsverstärker 130 wird eine Spannung 42 erzeugt, die der Differenz $B_2 - B_1$ entspricht und die im Dividierverstärker 135 durch die Spannung 41 dividiert wird, was eine dem Quotienten $(B_2 - B_1) / B_2$ proportionale Spannung 43 liefert.

Der Komparator 140 vergleicht nun die Spannung 43 mit einem Sollwert 44 der Spannungsquelle 145 und steuert im Alarmfall eine Alarmschaltung 150 an.

Für eine Auswerteschaltung 3, die nach Gl. (2) arbeiten soll, wird der Dividierverstärker 135 nicht benötigt und ein Verstärker 115 eingesetzt, der eine Spannung 39 erzeugt, die proportional zum Logarithmus der Intensität I des Messstrahls 71 ist.

Ist der erfindungsgemässe Gasdetektor 100 zum Nachweis von mehreren Gasen mit mehreren Infrarotstrahlungsdetektoren 90 bis 94 ausgerüstet, so wird für jedes Gas eine in Fig. 11 durch eine gestrichelte Linie abgetrennte Subschaltung 300 verwendet.

In den vorstehenden Beispielen ist das erfindungsgemässe Verfahren vorzugsweise am Nachweis von Kohlenmonoxid beschrieben. In gleicher Weise kann das Verfahren jedoch auch zum Nachweis von anderen reduzierenden Gasen, wie Aethan, Aethen, Aethin oder anderen Kohlenwasserstoffen oder Stickstoffoxid angewendet werden. Ein bevorzugter Anwendungsbereich ist in Geräten zur Ueberwachung der Umweltverschmutzung, insbesondere in Siedlungsballungebieten, wobei bei Ueberschreitung der Konzentration an bestimmten Schadstoffen Smog-Alarm in bestimmten Abstufungen ausgelöst werden kann.

0157243

## PATENTANSPRUECHE

1. Verfahren zum Nachweis von reduzierenden Gasen, vorzugsweise von Kohlenmonoxid in einem Gasgemisch, insbesondere Luft, durch Bestimmung der Intensitätsschwächung eines Infrarotstrahls in einem eine Infrarotstrahlungsquelle (60), einen Infrarotstrahlungsdetektor (90) une eine elektrische Auswerteschaltung (3) aufweisenden Gasdetektor (100), dadurch gekennzeichnet, dass ein Gasdetektor (100) verwendet wird, der eine gegen die Aussenatmosphäre abgeschlossene, einen Luftbewegungsgenerator (4) aufweisende Referenzkammer (5) und eine mit der Referenzkammer (5) über mindestens eine Verbindungsöffnung (8) in Verbindung stehende, eine Einlassöffnung (9) zum Einlass des zu untersuchenden Gasgemisches und einen durch eine Heizung (10) auf eine bestimmte Temperatur erhitzbaren, in einer Katalysaatorschicht (14) mindestens ein Uebergangsmetall aus einer der Gruppen I, II, VII und VIII des Periodensystems der chemischen Elemente mit einem Atomgewicht zwischen 100 und 205 enthaltenden Gassensor (11) aufweisende Messkammer (7) aufweist, dass das auf reduzierende GAse zu untersuchende Gasgemisch periodisch während einer Einsaugphase (28) durch Volumenvergrösserung der Referenzkammer (5) mittels des Luftbewegungsgenerators (4) durch die Einlassöffnung (9) in die Messkammer (7) und mindestens teilweise durch diese hindurch in die Referenzkammer (5) gesaugt wird, wobei die Temperatur des Gassensors (11) während eines Teils der Einsaugphase (28) so hoch ist, dass gegebenenfalls vorhandene reduzierende Gase oxidiert werden und während des Rests der Einsaugphase (28) so niedrig ist, dass vorhandene reduzierende GAse am Gassensor (11) adsorbiert werden, so dass ein Referenzgas mit keinem oder einem geringeren Gehalt an reduzierenden Gasen durch die Verbindungsöffnung (8) in die Referenzkammer (5) gelangt, dass das Referenzgas periodisch während einer Ausblasphase (29) durch Volumenverminderung der Referenzkammer (5) mittels des Luftbewegungsgenerators (4) durch die Messkammer (7) hindurch durch die Einlassöffnung (9) ausgeblasen wird, wobei die Temperatur des Gassensors (11) während eines Teils der Ausblasphase (29) annähernd die gleiche Temperatur aufweist, bei der während der Einsaugphase (28) Oxidation der reduzierenden Gase erfolgte und während des Rests der Ausblasphase (29) annähernd die Temperatur aufweist, bei der während der Einsaugphase (28) Adsorption der reduzierenden Gase erfolgte, dass die Intensität eines von der Infrarotstrahlungsquelle (60) ausgehenden Infrarotstrahls (69), der durch den Gassensor (11) hindurchtritt und dann als Messstrahl (71) auf den Infrarotstrahlungsdetektor (90) fällt, in der elektrische Auswerteschaltung (3) ermittelt wird, dass ein Wert ($B_1$) der Intensität des Messstrahls (71) während der Zeit der Einsaugphase (28) gemessen wird, in welcher Zeit der Gassensors (11) eine Temperatur aufweist, bei der reduzierende Gase adsorbiert werden und ein Wert ($B_2$) der Intensität des Messstrahls (71) während der Zeit der Ausblasphase (29) gemessen wird, in welcher Zeit der Gassensor (11) eine Temperatur aufweist, bei der reuduzierende Gase adsorbiert werden und dass die beiden Werte ($B_1$, $B_2$) in vorbestimmter Relation zum Nachweise von reduzierenden Gasen ausgewertet werden.

2. Verfahren gemäss Patentanspruch 1, dadurch gekennzeichnet, dass die Länge der aus einer Einsaugphase (28) und einer Ausblasphase (29) bestehenden Gasaustauschperiode (27) so gewählt wird, dass sie mit der doppelten Länge des Temperaturzyklus übereinstimmt, wobei die Temperatur des Gassensors (11) während eines Oxidationstemperaturzeitabschnitts in der Einsaugphase (28) auf einer Temperatur, bei der Oxidation von reduzierenden Gasen an der Katalysatorschicht (14) des Gassensors (11) erfolgen kann, vorzugsweise auf etwa 150°C, während eines Adsorptionstemperaturzeitabschnitts in der Einsaugphase (28) auf einer niedrigeren Temperatur, bei der Adsorption von reduzierenden Gasen an der Katalysatorschicht (14) des Gassensors (11) erfolgen kann, vorzugsweise auf etwa 30°C, gehalten wird, während eines Oxidationstemperaturzeitabschnitts in der Ausblasphase (29) annähernd auf der gleichen Oxidationstemperatur wie in der Einsaugphase (28) und während eines Adsorptionstemperaturzeitabschnitts in der Ausblasphase (29) annähernd auf der gleichen Adsorptionstemperatur wie in der Einsaugphase (28) gehalten wird und dass in der Auswerte-schaltung (3) der Quotient $(B_2-B_1)/B_2$ der Differenz des während des Adsorptionstemperaturzeitabschnitts in der Ausblasphase (29), vorzugsweise am Ende desselben, gemessenen Werts $(B_2)$ minus des während des Adsorptionstemperaturzeitabschnitts in der Einsaugphase (28), vorzugsweise am Ende desselben, gemessenen Werts $(B_1)$ der Intensität des Messstrahls (71) dividiert durch den während des Adsorptionstemperaturzeitabschnitts in der Ausblasphase (29), vorzugsweise am Ende desselben, gemessenen Wert $(B_2)$ der Intensität des Messstrahls (71) gebildet wird und zur Bestimmung der Konzentration an reduzierenden Gasen ausgewertet wird.

3. Verfahren gemäss Patentanspruch 1, dadurch gekennzeichnet, dass die Länge der aus einer Einsaugphase (28) und einer Ausblasphase (29) bestehenden Gasaustauschperiode (27) so gewählt wird, dass sie nicht mit der doppelten Länge des Temperaturzyklus übereinstimmt, wobei die Länge der Gasaustauschperiode (27) vorzugsweise kürzer ist als die doppelte Länge des Temperaturzyklus.

0157243

4. Verfahren gemäss einem der Patentansprüche 1 bis 3, dadurch gekennzeichnet, dass in der Auswerteschaltung (3) ein Alarmsignal ausgelöst wird, wenn die Konzentration an einem reduzierenden Gas einen vorbestimmten Wert überschreitet.

5. Verfahren gemäss einem der Patentansprüche 1 bis 4, dadurch gekennzeichnet, dass in der Auswerteschaltung (3) ein Alarm-Signal ausgelöst wird, wenn die Differenz $(B_2-B_1)$ des während des Adsorptionstemperaturzeitabschnitts in der Ausblasphase (29), vorzugsweise am Ende desselben, gemessenen Werts $(B_2)$ minus des am während des Adsorptionstemperaturzeitabschnitts in der Einsaugphase (28), vorzugsweise am Ende desselben, gemessenen Werts $(B_1)$ der Intensität des Messstrahls (71) oder der Quotient $(B_2-B_1)/B_2$ der Differenz des während des Adsorptionstemperaturzeitabschnitts in der Ausblasphase (29), vorzugsweise am Ende desselben, gemessenen Werts $(B_2)$ minus des während des Adsorptionstemperaturzeitabschnitts in der Einsaugphase (28), vorzugsweise am Ende desselben, gemessenen Werts $(B_1)$ der Intensität des Messstrahls (71) dividiert durch den während des Adsorptionstemperaturzeitabschnitts in der Ausblasphase (29), vorzugsweise am Ende desselben, gemessenen Wert $(B_2)$ der Intensität des Messstrahls (71) oder der Ausdruck $- \log (B_1/B_2)$ aus dem während des Adsorptionstemperaturzeitabschnitts in der Einsaugphase (28), vorzugsweise am Ende desselben, gemessenen Wert $(B_1)$ und dem während des Adsorptionstemperaturzeitabschnitts in der Ausblasphase (29), vorzugsweise am Ende desselben, gemessenen Wert $(B_2)$ der Intensität des Messstrahls (71) einen vorbestimmten Wert überschreitet.

0157243

6. Vorrichtung zur Durchführung des Verfahrens gemäss einem der Patentansprüche 1 bis 5, gekennzeichnet durch einen Gasdetektor (100), der eine gegen die Aussenatmosphäre abgeschlossene, einen Luftbewegungsgenerator (4) aufweisende Referenzkammer (5) und eine mit der Referenzkammer (5) über mindestens eine Verbindungsöffnung (8) in Verbindung stehende, eine Einlassöffnung (9) zum Einlass des zu untersuchenden Gasgemisches und einen durch eine Heizung (10) auf eine bestimmte Temperatur erhitzbaren, in einer Katalysatorschicht (14) mindestens ein Uebergangsmetall aus einer der Gruppen I, II, VII und VIII des Periodensystems der chemischen Elemente mit einem Atomgewicht zwischen 100 und 205 enthaltenden Gassensor (11) aufweisende Messkammer (7) aufweist.

7. Vorrichtung gemäss Patentanspruch 6, dadurch gekennzeichnet, dass der Gasdetektor (100) im Gassensor (11) eine Katalysatorschicht (14) aufweist, die mindestens eines der Metalle Rhodium, Rhenium, Iridium, Platin, Palladium, Osmium, Ruthenium, Silber oder Gold enthält.

8. Vorrichtung gemäss einem der Patentansprüche 6 und 7, dadurch gekennzeichnet, dass das Metall, bzw. die Metalle in der Katalysatorschicht (14) in feinstverteilter Form vorliegen.

9. Vorrichtung gemäss einem der Patentansprüche 6 und 7, dadurch gekennzeichnet, dass das Metall, bzw. die Metalle in der Katalysatorschicht (14) in Form eines die Infrarotstrahlung reflektierenden dünnen Films vorliegen.

10. Vorrichtung gemäss einem der Patentansprüche 6 bis 8, dadurch gekennzeichnet, dass das Metall, bzw. die Metalle in der Katalysatorschicht (14) in feinstverteilter Form auf einen Träger aufgebracht ist, bzw. aufgebracht sind, vorzugsweise auf einem Träger, der mindestens ein Oxid von Magnesium, Calcium, Strontium, Barium, Lanthan, Cer, Thorium, Titan, Zirkon, Vanadium, Niob, Tantal, Chrom, Molybdän, Wolfram, Mangan, Eisen, Kobalt, Nickel, Kupfer, Zink, Cadmium, Aluminium, Indium, Silicium, Zinn, Blei, Antimon oder Wismut enthält.

11. Vorrichtung gemäss einem der Patentansprüche 6 bis 10, dadurch gekennzeichnet, dass der Gasdetektor (100) elektrisch oder mechanisch wirkende Elemente aufweist, die die Infrarotstrahlungsquelle (60) und/oder den Infrarotstrahl (69) und/oder den Messstrahl (71) modulieren und dass Schaltelemente für eine phasenempfindliche Detektion vorhanden sind.

12. Vorrichtung gemäss einem der Patentansprüche 6 bis 11, dadurch gekennzeichnet, dass der Gasdetektor (100) zum Nachweis von Kohlenmonoxid in Strahlungsrichtung hinter der Infrarotstrahlungsquelle (60) und/oder vor dem Strahlungsdetektor (90) ein Infrarotfilter (80) mit einem Durchlässigkeitsbereich von 1900 bis 2200 $cm^{-1}$ aufweist.

13. Vorrichtung gemäss einem der Patentansprüche 6 bis 12, dadurch gekennzeichnet, dass Messkammer (7), Verbindungsöffnung (8) und Referenzkammer (5) des Gasdetektors (100) annähernd gleichen Querschnitt aufweisen, so dass sie unmittelbar ineinander übergehen.

14. Vorrichtung gemäss einem der Patentansprüche 6 bis 13, dadurch gekennzeichnet, dass der Gasdetektor (100) als Luftbewegungsgenerator (4) eine elektromagnetisch, elektrostatisch, piezoelektrisch oder thermomechanisch erregbare Membran in der Art eines Lautsprechers, eine Piezofolie aus PVDF, ein bimorphes piezoelektrisches oder bimetallisches Element oder eine durch Mikrolithographie erzeugte dünne Siliziumfolie aufweist.

15. Vorrichtung gemäss einem der Patentansprüche 6 bis 14, dadurch gekennzeichnet, dass der Gasdetektor (100) in dem Infrarotstrahlengang einen Hohlspiegel (31) aufweist, der die von der Infrarotstrahlungsquelle (60) ausgehende Infrarotstrahlung so reflektiert, dass sie nach der Reflektion an der unter der Katalysatorschicht (14) befindlichen reflektierenden Schicht (20) auf den Infrarotstrahlungsdetektor (90) fokussiert wird.

16. Vorrichtung gemäss einem der Patentansprüche 6 bis 15, dadurch gekennzeichnet, dass der Gasdetektor (100) zum Nachweis von Kohlenmonoxid und mindestens einer weiteren Gaskomponente mindestens eine weitere Katalysatorschicht (15), die auf weitere Gaskomponenten empfindlich ist, enthält, wobei die von der Infrarotstrahlungsquelle (60) ausgehende Infrarotstrahlung durch einen ersten Hohlspiegel (31) auf die erste, auf Kohlenmonoxid empfindliche Katalysatorschicht (14) und durch mindestens einen weiteren Hohlspiegel (32) auf mindestens eine weitere Katalysatorschicht (15), die auf eine weitere Gaskomponente empfindlich ist, reflektiert wird und wobei die von den reflektierenden Schichten (20, 21) reflektierten Messstrahlen (71, 72) auf unterschiedliche Infrarotstrahlungsdetektoren (90, 91) fokusiert werden.

17. Vorrichtung gemäss Patentanspruch 12 und 16, dadurch gekennzeichnet, dass der Gasdetektor (100) zum Nachweis von Kohlenmonoxid eine Katalysatorschicht (14) aus Rhodium auf Aluminiumoxid und in Strahlungsrichtung hinter der Infrarotstrahlungsquelle (60) und/oder vor dem Strahlungsdetektor (90) ein Infrarotfilter (80) mit einem Durchlässigkeitsbereich von 1900 bis 2200 cm$^{-1}$ aufweist und zum Nachweis von Aethin eine Katalysatorschicht (15) aus Palladium auf Siliziumdioxid und ein Infrarotfilter (81) mit einem Durchlässigkeitsbereich von 2950 bis 3150 cm$^{-1}$ oder zum Nachweis von Aethen eine Katalysatorschicht (15) aus Platin auf Siliziumdioxid und ein Infrarotfilter (81) mit einem Durchlässigkeitsbereich von 2750 bis 3050 cm$^{-1}$ aufweist.

18. Vorrichtung gemäss einem der Patentansprüche 6 bis 14, dadurch gekennzeichnet, dass der Gasdetektor (100) einen Gassensor (11) aufweist, bei dem die unter der Katalysatorschicht (14) angeordnete reflektierende Schicht (20) als Hohlspiegel in der Weise ausgebildet ist, dass die von der Infrarotstrahlungsquelle (60) ausgehende Infrarotstrahlung auf den Infrarotstrahlungsdetektor (90) fokussiert wird.

0157243

19. Vorrichtung gemäss einem der Patentansprüche 6 bis 15, dadurch gekennzeichnet, dass der Gasdetektor (100) eine langgestreckte Infrarotstrahlungsquelle (61) aufweist, dass der Infrarotstrahl (69) nach Reflexion an dem Hohlspiegel (31) auf die langgestreckte Katalysatorschicht (14) fällt und nach Reflexion an der reflektierenden Schicht (20) durch die einen unterschiedlichen Durchlässigkeitsbereich aufweisenden Infrarotfilter (80 bis 84) hindurch auf die langgestreckt nebeneinander angeordneten, breitbandigen Infrarotstrahlungsdetektoren (90 bis 94) fokussiert wird und dass die von den Infrarotdetektoren (90 bis 94) abgegebenen elektrischen Signale in der Auswerteschaltung (3) zum Nachweis verschiedener Gaskomponenten in dem zu untersuchenden Gasgemisch ausgewertet werden.

20. Vorrichtung gemäss einem der Patentansprüche 6 bis 14, dadurch gekennzeichnet, dass der Gasdetektor (100) eine Infrarotstrahlungsquelle (60) aufweist, deren Infrarotstrahl (69) durch den Gassensor (11) hindurch auf den Infrarotstrahlungsdetektor (90) fällt.

21. Vorrichtung gemäss Patentanspruch 19, dadurch gekennzeichnet, dass sich im Strahlengang des Infrarotstrahls (69) eine Infrarotlinse (50) befindet, die den Infrarotstrahl (69), bzw. den Messstrahl (71) auf den Infrarotstrahlungsdetektor (90) fokussiert, wobei vorzugsweise die Infrarotlinse (50) und das Infrarotfilter (80) als Infrarotfenster (85) der Messkammer (7) dienen.

22. Vorrichtung gemäss einem der Patentansprüche 6 bis 14 und 19, dadurch gekennzeichnet, dass der Gasdetektor (100) einen Hohlspiegel (31) aufweist, der den Infrarotstrahl (69) der Infrarotstrahlungsquelle (60), bzw. den Messstrahl (71) durch den Gassensor (11) hindurch auf den Infrarotstrahlungsdetektor (90) fokussiert.

23. Vorrichtung gemäss einem der Patentansprüche 6 bis 14, dadurch gekennzeichnet, dass der Gasdetektor (100) einen ersten Hohlspiegel (31), der den Infrarotstrahl (69) der Infrarotstrahlungsquelle (60) durch das Infrarotfenster (85) hindurch auf den Gassensor (11) fokussiert, und einen zweiten Hohlspiegel (32), der den vom Gassensor (11) ausgehenden Messstrahl (71) durch das Infrarotfilter (80) hindurch auf den Infrarotstrahlungsdetektor (90) fokussiert, aufweist.

24. Vorrichtung gemäss einem der Patentansprüche 6 bis 23, dadurch gekennzeichnet, dass die Einlassöffnung (9) des Gasdetektors (100) als Kapillare ausgebildet ist, deren Länge vorzugsweise mindestens 2 mm und deren Innendurchmesser vorzugsweise höchstens 1 mm, insbesondere etwa 0,3 mm beträgt.

_Fig. 1_

Fig. 2

0157243

Fig. 3

_Fig. 4_

31
69
60

71

85
14
20
10
5
4

_11_

12
90
80
9
7

3

_100_

4 (11)

0157243

**Fig. 5**

Fig.6

*Fig. 7*

9  10  7  85  80  90

100

60

69
85
10

71

11

14

5

4

12

3

7 (11)

0157243

*Fig. 8*

69  50  14        9 10 7 80 90

60                        12        <u>100</u>

<u>11</u>

71

10

<u>5</u>
4

3

8 (11)

0157243

0157243

9 (11)

Fig. 9

Fig. 10

Fig. 11